# NEW EUROPEAN PATENT SPECIFICATION

(11) **EP 2 758 514 B2**
(45) Date of publication and mention of the opposition decision: **18.03.2020**
(45) Mention of the grant of the patent: 22.02.2017
(21) Application number: 12766564.4
(22) Date of filing: 20.09.2012
(51) Int. Cl.: C12N 1/08, C12N 1/14, C12P 21/02, C12N 9/16, C12N 9/20, C12Q 1/68

(54) **ENDOGENOUS DNASE ACTIVITY TO REDUCE DNA CONTENT**
ENDOGENE DNASE-WIRKUNG ZUR REDUZIERUNG EINES DNA-INHALTS
ACTIVITÉ DNASE ENDOGÈNE POUR RÉDUIRE LA TENEUR EN ADN

(30) Priority: 22.09.2011 US 201161537837 P
(43) Date of publication of application: 30.07.2014
(73) Proprietor: Danisco US Inc., Palo Alto, California 94304 (US)
(72) Inventor: HOFFMANN, Katherine, Palo Alto, California 94304 (US); KO, Douglas, Palo Alto, California 94304 (US); WARD, Michael, Palo Alto, California 94304 (US)
(74) Representative: Mewburn Ellis LLP
(86) International application number: PCT/US2012/056315
(87) International publication number: WO 2013/043860

(56) References cited:
- WO-A1-2008/065200
- WO-A1-2008/065200
- WIEBE ET AL: "Stable production of recombinant proteins in filamentous fungi - problems and improvements", MYCOLOGIST, CAMBRIDGE UNIVERSITY PRESS, CAMBRIDGE, GB, vol. 17, no. 3, 1 August 2003 (2003-08-01) , pages 140-144, XP022293489, ISSN: 0269-915X, DOI: 10.1017/S0269915X03003033
- KERÄNEN ET AL: "Production of recombinant proteins in the filamentous fungus Trichoderma reesei", CURRENT OPINION IN BIOTECHNOLOGY, LONDON, GB, vol. 6, no. 6, 1 January 1995 (1995-01-01) , pages 534-537, XP002109010, ISSN: 0958-1669, DOI: 10.1016/0958-1669(95)80088-3
- SISNIEGA HEIDI ET AL: "Strategies for large-scale production of recombinant proteins in filamentous fungi", METHODS IN BIOTECHNOLOGY HUMANA PRESS INC, 999 RIVERVIEW DR, STE 208, TOTOWA, NJ 07512-1165 USA SERIES : METHODS IN BIOTECHNOLOGY, 2005, pages 225-238, XP008158063,
- COOKE G D ET AL: "A modified Escherichia coli protein production strain expressing staphylococcal nuclease, capable of auto-hydrolysing host nucleic acid", JOURNAL OF BIOTECHNOLOGY, ELSEVIER SCIENCE PUBLISHERS, AMSTERDAM, NL, vol. 101, no. 3, 20 March 2003 (2003-03-20), pages 229-239, XP002473877, ISSN: 0168-1656, DOI: 10.1016/S0168-1656(02)00339-5

## Description

### BACKGROUND

Filamentous fungi *(e.g., Trichoderma, Aspergillus, Geosmithia, Myceliophthora, Penicillium, Fusarium, Humicola,* and others) have become popular host strains for protein production in recent years. Enzyme preparations produced by fungal species such as *Trichoderma reesei, Aspergillus niger, Aspergillus tubingensis, Aspergillus oryzae, Geosmithia emersonii, Mycellophthora thermophila, Pencillium funiclosum, Fusarium venenatum*, and *Humicola insolens* have been developed as commercial products. Filamentous fungi, such as *Trichoderma, Aspergillus, Myceliophthora, Penicillium, Fusarium*, and others, have also been engineered to express heterologous proteins, *e.g.,* enzymes and therapeutic proteins, typically under control of inducible promoters (see, *e.g.,* England *et al*., PCT Patent publication WO2004/035070). Many thus prepared fungal (*e.g., T. reesei, A. niger, A. tubingensis, A. oryzae, G. emersonii, Ivf. thermophila, P. funiculosum, F. venenatum,* and *H. insolens*) proteins are useful as food or feed additives (see, *e.g.,* Dunn-Coleman *et al*., in PCT patent publication WO2003/038035) or in other industrial applications. Because production of proteins in fungi is usually carried out on a large scale, improvements in production and processing efficiency can have great economic significance.
Wiebe et al. (Mycologist, 17(3): 140-144, 2003) describes the problems affecting recombinant protein production in filamentous fungi.
Keranen et al. (Current Opinion in Biotechnology, 8(6):534-537, 1995) relates to the production of heterologous proteins in the filamentous fungus *Trichoderma reesei* using experiments in which antibody Fab fragments were expressed.
Sisniega et al. (Methods in Biotechnology, 18:225-238, 2005) describes strategies for large-scale production of recombinant proteins in filamentous fungi.

### BRIEF DESCRIPTION OF THE FIGURES

Figure 1: Effect of elevated pH or temperature on DNA degradation observed from an ultrafiltration concentrate of a fermentation broth of a *T. reesei* expressing a phytase from *Buttiauxella* sp.
Figure 2: Effect of incubation time on DNA degradation observed from the same ultrafiltration concentrate as above of a fermentation broth of a *T. reesei* expressing a phytase from *Buttiauxella* sp.
Figure 3: Effect of elevated temperatures and incubation time on DNA degradation observed from the same ultrafiltration concentrate as above of a fermentation broth of a *T*. *reesei* expressing a phytase from *Buttiauxella* sp.
Figures 4A-4B: Effects of elevated pH on DNA degradation observed at pH levels 5.2, 6.3, and 7.6, observed from an ultrafiltration concentrate of a fermentation broth of a *T. reesei* expressing a lipase from *Aspergillus tubingensis.*

### SUMMARY

Accordingly, the present invention provides a method of reducing DNA content of a broth in which filamentous fungal host cells has been cultured, comprising the steps of:
performing a solid-liquid separation step to separate the broth from filamentous fungal host cells before adjusting the pH and/or temperature of a broth in which the fungal host cells have been cultured for at least 24 hours to increase the pH and/or the temperature used in culturing;
incubating the broth for a sufficient period at the increased pH and/or temperature to detectably reduce fungal host DNA in the preparation; and assessing the DNA content of the broth before and after the incubating step;
wherein the reduction of DNA content is primarily due to the presence of endogenous DNase in the broth.
In some methods, the broth is at room temperature after the ultrafiltration step. In some methods, the temperature of the broth before the adjusting step is 25°C to 34° C. In some methods, the pH of the broth before the adjusting step is between 4 and 5. Some methods further comprise culturing the filamentous fungal host cells in the broth until a desired concentration of secreted proteins of interest in the broth is obtained before the adjusting step. Preferably, the pH, and/or temperature adjusting step is performed before the enzyme(s) produced by the host cell is/are applied to treat or act on an intended substrate.

In some methods, the pH is increased to pH 6-8 during the adjusting step. In some methods, the temperature is increased to 35° C-47° C during the adjusting step. The methods comprise assessing the DNA content of the broth. In the methods, the DNA content is assessed before and after the incubating step. In some methods, the DNA content is reduced to an undetectable level as assessed by PCR and/or gel electrophoresis with ethidium bromide staining. Some methods further comprise allowing the broth to cool to room temperature after the incubating step. Some methods further comprise purifying one or more proteins from the broth. In some methods, one or more proteins in the broth is/are recombinantly expressed by the filamentous fungal host cell. In some methods, the filamentous fungal host cells lack an exogenous DNase. In some methods, no DNase is added to the broth.
In some methods, the filamentous fungal host cells recombinantly express a cellulase enzyme.
In some methods, the filamentous fungal host cells recombinantly express a phytase. In some methods, the filamentous fungal host cells recombinantly express a lipase.

In any of the above methods, the fungal host cell can be a cell of *T. reesei, A. niger, A. tubingensis, A. oryzae, G. emersonii, M. thermophila, P. funiculosum, F. venenatum,* or *H. insolens.*

### DEFINITIONS

A "DNase" is an enzyme capable of degrading DNA, usually by cleaving of a phosphodiester bond. DNases include endonucleases that cleave internal sites and exonucleases that cleave mononucleotides from the end of a DNA molecule. DNases are usually proteins but can also be non-protein DNases. DNases may or may not have RNase activity as well as DNase activity.

An "exogenous" protein (*e.g.*, an exogenous DNase) refers to a protein introduced into a host strain by recombinant expression or added to extracts of the host strain from an external supply of the protein. An exogenous protein can be heterologous to the host strain (*i*.*e*., naturally produced by a different host strain) or homologous (*i*.*e*., naturally produced by the host strain).

The term "recombinant" refers to a polynucleotide or polypeptide that does not naturally occur in a host cell. A recombinant molecule may contain two or more naturally occurring sequences that are linked together in a way that does not occur naturally.

The term "heterologous" refers to elements that are not normally associated with each other. For example, if a host cell produces a heterologous protein, that protein is not normally produced in that host cell. Likewise, a promoter that is operably linked to a heterologous coding sequence is a promoter that is operably linked to a coding sequence that it is not usually operably linked to in a wild-type host cell. The term "homologous" with reference to a polynucleotide (*e*.*g*., a DNA) or protein, refers to a nucleic acid (*e.g.,* a DNA) or protein that occurs naturally in a host cell.

A "gene" refers to a DNA segment that is involved in producing a polypeptide and includes regions preceding and following the coding segments(s) (exons) and in some genes intervening segments (introns) between individual coding segments.

Nucleic acids include DNA, RNA, single-stranded or double-stranded and chemically modified versions thereof.

A "vector" is a polynucleotide sequence designed to introduce nucleic acids into one or more cell types. Vectors include cloning vectors, expression vectors, shuttle vectors, plasmids, phage particles, cassettes and the like.

A "phytase" is an enzyme that catalyzes the hydrolysis of phytate to (1) myo-inositol and/or (2) mono-, di-, tri-, tetra- and/or penta-phosphates thereof and (3) inorganic phosphate. For example, phytases include enzymes defined by EC number 3.1.3.8, or EC number 3.1.3.26.

"Cellulase enzymes" or "cellulases" include enzymes that act on cellulose directly and accessory enzymes that facilitate the direct action of other enzymes on cellulose. Cellulases include bacterial or fungal exoglucanases or exocellobiohydrolases, and/or endoglucanases, and/or β-glucosidases. These three different types of cellulase enzymes act synergistically to convert cellulose and its derivatives to glucose. Cellulase enzymes also include accessory enzymes, including GH61 members, such as EG4, swollenin, loosenin, CIP1, and the like.

A "lipase" is an enzyme that catalyzes the hydrolysis or formation of lipids. For example, lipases catalyze the hydrolysis of triacylglycerol to produce diacylglycerol and carboxylate. Lipases include enzymes defined by EC number 3.1.1.3.

"Isolated" means an object specifies is removed from at least one component with which it is naturally associated.

"Purified" means that an object species is at least 50% (w/w), and sometimes at least 75, 90, 95 or 99% (w/w) free of macromolecular contaminants used in its production or purification but does not exclude the presence of excipients added to facilitate the use of the object species.

A protein preparation includes one or more desired proteins secreted by a filamentous fungal host (*e.g., T reesei, A. niger, A. tubingensis, A. oryzae, G. emersonii, M. thermophila, P. funiculosum*, *F. venenatum*, *H. insolens* and others) in any state of purity and may also include contaminants from production or purification of the desired proteins(s) or added excipients. Thus, a protein preparation can be a broth or protein(s) purified from a broth.

A broth or a "fermentation broth" refers to culture media used for culturing a filamentous fungal host cell (*e.g., T. reesei*, *A. niger, A. tubingensis, A. oryzae, G. emersonii, M. thermophila*, *P. funiculosum*, *F. venenatum*, and *H. insolens*) with or without removal of cells and cell debris after fermentation, and with or without concentration of proteins and other macromolecules in the broth by ultrafiltration or similar technique but does not include preparation of purified proteins in which the desired protein(s) have been separated from broth by techniques such as protein precipitation and resuspension in a fresh medium or column chromatography and elution in a fresh medium.

The term "filamentous fungi" refers to all filamentous forms of the subdivision Eumycotina(See, Alexopoulos, C. J. (1962), INTRODUCTORY MYCOLOGY, Wiley, New York and AINSWORTH AND BISBY DICTIONARY OF THE FUNGI, 9.sup.th Ed. (2001) Kirk et al., Eds., CAB International University Press, Cambridge UK). These fungi are characterized by a vegetative mycelium with a cell wall composed of chitin, cellulose, and other complex polysaccharides. The filamentous fungi of the present invention are morphologically, physiologically, and genetically distinct from yeasts. Vegetative growth by filamentous fungi is by hyphal elongation and carbon catabolism is obligatorily aerobic.

The term "*Trichoderma*" or "*Trichoderma* sp." refer to any fungal genus previously or currently classified as "*T*." and hybrids of such strains, as well as genetically modified forms thereof (*e*.*g*., modified by mutation or a transgene, or a gene knockout).

A "feed" means any natural or artificial diet, meal or the like or components of such meals intended or suitable for being eaten, taken in, digested, by a non-human animal.

A "food" means any natural or artificial diet, meal or the like or components of such meals intended or suitable for being eaten, taken in, digested, by a human being.

A "food or feed additive" is a purified compound or a multi component composition intended for or suitable for being added to food or feed. It may include one or more compounds such as vitamins, minerals, enzymes and suitable carriers and/or excipient.

Terms such as "assess," "measure," or "determine" encompass qualitative or quantitative detection of an analyte, particularly an endogenous DNA. Such assessment or determination may thus indicate presence or absence of an analyte or an amount of the analyte.

The term "comprising" and its cognates are used in their inclusive sense; that is, equivalent to the term "including" and its corresponding cognates.

Unless otherwise apparent from the context, Reference to a specific numeric value encompasses the stated value and such variation as is inherent in its measurement (*i*.*e*., +/-SEM).

Unless otherwise apparent from the context, "about" indicates a tolerance of +/- 10%.

Numeric ranges are inclusive of the numbers defining the range. Some preferred subranges are also listed, but in any case, reference to a range includes all subranges defined by integers included within a range.

### DETAILED DESCRIPTION

### I. General

The present application provides a method of reducing the DNA content of a protein preparation from filamentous fungi without the need to use an exogenous DNase. The application is based in part on the observation of an endogenous filamentous fungal DNase activity in culture broths together with commercially valuable proteins. For example, DNase activity is found in the culture broth of a *T. reesei*, *A. niger, A. tubingensis, A. oryzae, G. emersonii*, *M. thermophila*, *P. funiculosum*, *F. venenatum*, *H. insolens*, or another strain expressing and/or producing certain industrial enzyme(s) of interest. Although an understanding of mechanism is not required for practice of the invention, it is believed that the endogenous DNase activity may have been the result of either one or more secreted DNases endogenous to the filamentous fungus, (*e.g., T. reesei, A. niger, A. tubingensis, A. oryzae, G. emersonii, M. thermophila*, *P. funiculosum*, *F. venenatum*, *H. insolens*, and so forth) or one or more intracellular DNases to such filamentous fungi being released due to cell lysis.

Such endogenous DNase activity can be used to reduce or eliminate DNA molecules from a filamentous fungal culture broth, for example, a *T*. *reesei*, *A. niger*, *A. tubingensis*, *A. oryzae, G. emersonii, M. thermophila*, *P. funiculosum*, *F. venenatum*, or *H. insolens* culture broth. Removal of such DNA molecules is useful in many applications, for example, in providing an enzyme preparation as a food or feed additive or supplement. Some commercial enzyme preparations, particularly those used in preparation of foods or feed, are required by regulatory authorities to be free of detectable host DNA or at least have host DNA below a defined limit. Although fungal DNA (*e.g.*, DNA from *T*. *reesei*, *A. niger*, *A. tubingensis*, *A. oryzae*, *G*. *emersonii, M. thermophila*, *P. funiculosum, F. venenatum*, *H. insolens*, and so forth) in culture broth could be removed with an exogenous DNase, expressing such a DNase recombinantly or supplying it to the culture broth involves additional steps, increased costs, and possibly reduced efficiency. The present method provides a simple procedure of removing host genetic materials in a protein preparation without the need of either genetically manipulating the host cell or adding DNase(s) into the preparation.

### II. Filamentous fungal host strains

Any suitable fungal host strains capable of expressing proteins of either heterologous or endogenous varieties can be used to practice the present invention. For example, fungal host strains can be host strains of filamentous fungal species, such as those from the phylum Ascomycota, and the subphylum Pezizomycotina. Such organisms include filamentous fungus cells used for the production of commercially important industrial and pharmaceutical proteins, including, but are not limited to *Trichoderma* spp., *Aspergillus* spp., *Fusarium* spp., *Penicillium* spp., *Chrysosporium* spp., *Talaromyces* spp., *Geosmithia* spp., *Myceliophthora* spp., and *Neurospora* spp. Particular organisms from which suitable host strains may be derived may include, but are not limited to, *Trichoderma reesei* (previously classified as *Trichoderma longibrachiatum* and *Hypocrea jecorina*), *Aspergillus niger, Aspergillus fumigatus*, *Aspergillus itaconicus*, *Aspergillus oryzae*, *Aspergillus nidulans*, *Aspergillus terreus*, *Aspergillus sojae*, *Aspergillus japonicus*. *Aspergillus tubingensis*, *Humicola insolens*, *Humicola grisea*, *Thermomyces lanuginosus*, *Neurospora crassa*, *Penicillium funiculosum*, *Penicillium chrysogenum*, *Talaromyces (Geosmithia) emersonii*, *Fusarium venenatum*, *Fusarium graminearum*, *Myceliophthora thermophila*, and *Chrysosporium lucknowense.* Fungal host strains can also be host strains of filamentous fungal species, such as those from the phylum Basidiomycota or the subphylum Mucormycotina. Such organisms include filamentous fungus cells used for the production of commercially important industrial and pharmaceutical proteins, including, but are not limited to *Agaricus* spp., *Phanerochaete* spp., *Schizophyllum* spp., *Rhizomucor* spp., and *Mucor* spp. Particular organisms from which suitable host strains may be derived may include, but are not limited to *Agaricus bisporus*, *Phanerochaete chrysosporium*, *Schizophyllum commune*, *Rhizomucor miehei*, and *Mucor circinelloides.*

It can be expected that some level of endogenous DNase activity will exist in fermentation products (*e*.*g*., a protein preparation, including, for example, a culture broth) derived from the above-listed and other species of filamentous fungi. The level of DNase activity and the pH or temperature optima of the DNase may vary between and among species. But the adjustments made to pH, temperature and time of incubation as described herein can be tested in order to determine the requirements for DNA removal, in accordance with the present disclosure.

In a particular embodiment of any one of the present methods, the host cell is a cell of *T*. *reesei,* a well-known filamentous fungus. Examples of *T*. *reesei* strains include ATCC No. 13631, ATCC No. 26921, ATCC No. 56764, ATCC No. 56765, ATCC No. 56767, and NRRL No. 15709. One example of a host cell is derived from the RL-P37 *T*. *reesei* strain (described in Sheir-Neiss et al. (1984) Appl. Microbiol. Biotechnology 20:46-53. Another host cell is the Morph 1.1 (pyr+) *T*. *reesei* strain, a spontaneous pyr4 revertant of the quad-deleted RL-P37 *T*. *reesei* strain (described in PCT Patent publication WO 05/001036). Other host strains similar to RL-P37 include *T*. *reesei* (longibrachiatum) strain RUT-C30 (ATCC No. 56765) and strain QM9414 (ATCC No. 26921).

In certain embodiments, the host strain may have been genetically manipulated through genetic engineering, classic mutagenesis, or by forming hybrids of existing strains. Genetic engineering can be used to introduce exogenous genes or knockout or knockdown endogenous genes. Mutagenesis can be used to inhibit or knockout endogenous genes or, in some host cells, change or enhance the function of endogenous genes. Examples include overproducing mutants as described in, *e.g.,* Bower *et al.*, PCT Patent publication WO2008/153903. Examples also include host strains in which various native genes of the fungal host cell have been inactivated. Gene inactivation may be accomplished by complete or partial deletion, by insertional inactivation, or by any other means, which renders a gene nonfunctional for its intended purpose (such that the gene is prevented from expression of a functional protein). Examples of methods for gene inactivation can be found in *e.g.,* U.S. Pat. Nos. 5,246,853 and 5,475,101, and PCT Patent Publication WO 92/06209. In some hosts, one or more genes encoding cellulolytic enzymes, such as endoglucanases (EG) and exocellobiohydrolases (CBH) (*e.g.*, *cbh1*, *cbh2*, *egl1*, or *egl2*) can be inactivated. For example, U.S. Pat. No. 5,650,322 discloses derivative strains of RL-P37 having deletions in both the *cbh1* gene and the *cbh2* gene. In a particular example, a "quad" deletion of *cbh1*, *cbh2*, *egl1* and *egl2* is described in U.S. Pat. No. 5,847,276 and PCT Patent publicationWO 05/001036. In yet a further example, certain host cells can be manipulated such that they are rendered protease-deficient or protease-minus strains, such that the risk of degradation of proteins of interest expressed by such strains is reduced or diminished.

As indicated, the present methods do not require supplementation of the host cell culture with an exogenous DNase, such as the procedures described by EP658621 or PCT Patent publication WO2008065200. However, host cells in which one or more exogenous DNases are recombinantly expressed, although not required, can be used, provided that the DNase activity employed in the method is not primarily that of the exogenous DNase(s). In some such host cells, the exogenous DNase is not expressed in active form (*e.g.,* it is expressed in inclusion bodies) or is only poorly expressed. In some such host cells, the exogenous DNase is not secreted extracellularly (*e.g.,* lacking a signal peptide). If any exogenous DNase exists in a culture broth or other protein preparation the contribution of such exogenous DNase to degradation of host DNA is no more than 49% (*e.g.*, no more than 49%, no more than 45%, no more than 40%, no more than 35%, no more than 30%, no more than 25%, no more than 20%, no more than 15%, no more than 10%, or no more than 5%) of the total DNase activity. Such can be demonstrated by showing that, under the conditions of temperature and pH of the assay (*e.g.,* pH 7.0, at a temperature of 40° C), the time taken to reduce endogenous DNA to an undetectable level is increased by no more than 49% (*e.g.*, no more than no more than 49%, no more than 45%, no more than 40%, no more than 35%, no more than 30%, no more than 25%, no more than 20%, no more than 15%, no more than 10%, or no more than 5%), and preferably increased by less than 25% or 10% in the absence than in the presence of the exogenous DNase(s). In other words, the endogenous DNase activity is primarily responsible for degrading the DNA of the host cell (*e.g., T. reesei*, *A. niger*, *A. tubingensis*, *A. oryzae*, *G. emersonii*, *M. thermophila, P. funiculosum*, *F. venenatum*, *H. insolens*, and so forth) in the culture broth or other protein preparation.

The host strain can be used for expression and preferably secretion of one or more endogenous or exogenous enzymes or a blend of endogenous and exogenous enzymes. Some examples of enzyme types that can be expressed endogenously or exogenously include amylolytic enzymes, proteolytic enzymes, cellulase enzymes, oxido-reductase enzymes and plant wall degrading enzymes. More specifically, such enzymes include amylases, proteases, xylanases, lipases, laccases, phenol oxidases, oxidases, cutinases, cellulases, hemicellulases, esterases, peroxidases, catalases, glucose oxidases, phytases, pectinases, glucosidases, isomerases, transferases, galactosidases and chitinases.

Alternatively or additionally, the host strain can be engineered to express and preferably secrete hormones, enzymes, growth factors, cytokines, antibodies and the like. Some examples of hormones that can be expressed include follicle-stimulating hormone, luteinizing hormone, corticotropin-releasing factor, somatostatin, gonadotropin hormone, vasopressin, oxytocin, erythropoietin, insulin and the like.

Growth factors are proteins that bind to receptors on the cell surface, with the primary result of activating cellular proliferation and/or differentiation. Some examples of growth factors to express include platelet-derived growth factor, epidermal growth factor, nerve growth factor, fibroblast growth factors, insulin-like growth factors, transforming growth factors and the like.

Cytokines are a unique family of growth factors. Secreted primarily from leukocytes, cytokines stimulate both the humoral and cellular immune responses, as well as the activation of phagocytic cells. Some examples of cytokines to express include colony stimulating factors, the interleukins (IL-1 a and β), IL-2 through IL-13) and the interferons (α, β, and y).

The host cells can also be engineered to express antibodies. Human, humanized, chimeric or veneered antibodies are preferred. Antibodies can be from any class and isotype, *i.e.*, G1, 2, 3 and 4, and A, M, E or D.

In certain embodiments, a nucleic acid segment encoding an exogenous protein is cloned into an expression vector. The nucleic acid segment encoding the exogenous protein can be placed in operable linkage with a signal peptide to confer secretion, or it can be placed in operable linkage with a promoter and sometimes other regulatory sequences for appropriate expression. An expression vector encoding a polypeptide can be transfected or transformed into a host cell using standard techniques as described by *e.g.,* Sambrook et al. (1989) Molecular Cloning: A Laboratory Manual, 2nd Edition, Cold Spring Harbor Laboratory Press. Nucleic acid can also be transferred into cells via a retroviral vector (*see e.g.,* Ferry et al. (1991) Proc. Natl. Acad. Sci., USA, 88: 8377-8381; and Kay et al. (1992) Human Gene Therapy 3: 641-647), an adenoviral vector (*see*, *e.g.*, Rosenfeld (1992) Cell 68: 143-155; and Herz and Gerard (1993) Proc. Natl. Acad. Sci., USA, 90:2812-2816), receptor-mediated DNA uptake (*see*, *e.g.,* Wu, and Wu (1988) J. Biol. Chem. 263:14621; Wilson et al. (1992) J. Biol. Chem. 267: 963-967; and U.S. Pat. No. 5,166,320), direct injection of DNA (*see*, *e.g.*, Acsadi et al. (1991) Nature 332: 815-818; and Wolff et al. (1990) Science 247:1465-1468) or particle bombardment (biolistics) (*see*, *e.g.*, Cheng et al. (1993) Proc. Natl. Acad. Sci., USA, 90:4455-4459; Zelenin et al. (1993) FEBS Letts. 315: 29-32).

Both episomal and integrating expression vectors can be used. To identify integtants from an integrating vector, a gene that contains a selectable marker (*e.g.,* drug resistance) is introduced into host cells along with the nucleic acid of interest. Examples of selectable markers include those that confer resistance to certain drugs, such as G418 and hygromycin. Selectable markers can be introduced on a separate vector from the nucleic acid of interest or on the same vector. Transfected host cells can then be identified by selecting for cells using the selectable marker. For example, if the selectable marker encodes a gene conferring neomycin resistance, host cells which have taken up nucleic acid can be identified by their growth in the presence of G418. Cells that have incorporated the selectable marker gene will survive, while the other cells die.

Once expressed, a polypeptide can be purified according to conventional procedures including affinity purification, ammonium sulfate precipitation, ion exchange chromatography, or gel electrophoresis (see generally, R. Scopes (1982) Protein Purification, Springer-Verlag, N.Y.; Deutscher (1990) Methods in Enzymology Vol. 182:Guide to Protein Purification, Academic Press, Inc. N.Y.). Alternatively, the polypeptide can be subject to minimal or no post expression manipulation such that it is used in a composition that is substantially similar to the culture broth of the host cells expressing it.

### III. Culturing a filamentous fungal host cell

Desired proteins such as cellulase enzymes, feed enzymes, or other enzymes, can be produced in cells of a filamentous fungal host (*e*.*g*., *T. reesei*, *A. niger*, *A. tubingensis*, *A. oryzae*, *G. emersonii, M*. *thermophila*, *P. funiculosum*, *F. venenatum*, *H. insolens*, and so forth) either by solid or submerged culture, including batch, fed-batch and continuous-flow processes. Fed-batch is widely used due to its ease of control, production of uniform quantities of products, and most economical uses of all equipment.

Culturing (sometimes referred to as fermentation) can be done in a liquid (*e*.*g*., an aqueous) medium, or in a solid medium, and the present method is applicable to either. Culturing is, in certain typical embodiments, performed in a broth including an aqueous mineral salts medium, organic growth factors, a carbon or energy source material, assimilable nitrogen, molecular oxygen, and a starting inoculum of the fungal host species to be employed. The mineral media suitably include certain amounts of phosphorus, magnesium, calcium, potassium, sulfur, and/or sodium, in soluble assimilable ionic and combined forms, and also preferably certain trace elements such as copper, manganese, molybdenum, zinc, iron, boron, and/or iodine, and others, again in suitable soluble assimilable form. The mineral nutrients can contribute to proper microorganism growth, maximizing the assimilation of the carbon and energy source by the cells in the microbial conversion process, and achieving maximum cellular yields.

The source of assimilable nitrogen can be any nitrogen-containing compound or compounds capable of releasing nitrogen in a form suitable for metabolic utilization by the microorganism. Although a variety of organic nitrogen source compounds, such as protein hydrolysates, can be employed, usually inexpensive nitrogen-containing compounds such as ammonia, ammonium hydroxide, urea, or various ammonium salts, such as ammonium phosphate, ammonium sulfate, ammonium pyrophosphate, ammonium chloride, or various other ammonium compounds can be utilized. Ammonia gas itself is convenient for large-scale operations, and can be bubbled through the aqueous ferment (fermentation medium) in suitable amounts. The ammonia can also assist in pH control.

Culturing is an aerobic process typically involving molecular oxygen supplied by a molecular oxygen-containing gas such as air, oxygen-enriched air, or even substantially pure molecular oxygen, provided to maintain the contents of the fermentation vessel with a suitable oxygen partial pressure effective in assisting the microorganism species to grow in a thriving fashion. In effect, by using an oxygenated hydrocarbon substrate, the oxygen requirement for growth of the microorganism is reduced. Nevertheless, molecular oxygen is supplied for growth, because the assimilation of the substrate and corresponding growth of the microorganisms, is, in part, a combustion process.

Although the aeration rate can vary over a considerable range, aeration generally is conducted at a rate that is in the range of about 0.5 to 10, preferably about 0.5 to 7, volumes (at the pressure employed and at 25° C) of oxygen-containing gas per liquid volume in the fermenter per minute. This amount is based on air of normal oxygen content being supplied to the reactor, and in terms of pure oxygen the respective ranges would be about 0.1 to 1.7, or preferably about 0.1 to 1.3, volumes (at the pressure employed and at 25° C) of oxygen per liquid volume in the fermenter per minute.

The pressure for fermentation can also range widely. Pressures generally are within the range of about 0 to 50 psig, preferably about 0 to 30 psig, more preferably at a level that is at least slightly over atmospheric pressure, as a balance of equipment and operating cost versus oxygen solubility may be achieved. Greater than atmospheric pressure is advantageous to increase dissolved oxygen concentration, which in turn can help increase cellular growth rates. However, higher pressure increases equipment and operating costs.

The fermentation temperature can vary somewhat. For example, for *T. reesei*, the temperature generally is within a range of about 20° C to about 40° C, generally preferably in the range of about 25° C to about 34° C. The preferred fermentation temperature for *T. reesei* is within the range of about 27° C to about 30°C.

The pH range in the aqueous microbial ferment (fermentation admixture) can, for example, be in the range of about 2.0 to about 8.0. With filamentous fungi, the pH normally is within the range of about 2.5 to about 8.0; for example, with *T*. *reesei*, the pH normally is within the range of about 3.0 to about 7.0. The preferred pH range for *T*. *reesei* is within the range of about 3.5 to about 5.0.

Although the average retention time of the fermentation admixture in the fermenter can vary considerably, depending in part on the fermentation temperature and culture employed, it is generally within a range of about 24 to about 500 hr, preferably 24 to 400 hr. In certain embodiments, the fungal host cell has been cultured for at least 24 hr, for example, for at least 48 hr, at least 72 hr, or at least 96 hr. For example, *T. reesei* has preferably been cultured for at least 24 hr, such as, *e.g.,* for at least 48 hr, at least 72 hr, or at least 96 hr. The fermentation is preferably continued until the cell density and/or concentration of one or more secreted proteins of interest is approaching a pre-determined desired level. Whether such a desired level has been reached can be determined using periodic sampling from the fermentation tank. The particular desired level of the cell density and/or concentration of one or more secreted protein of interest can vary and can be set in consideration of a number of factors. In certain embodiments, the particular desired level can be set at a point where the productivity of the host cell begins to decline. In certain other embodiments, the particular desired level can be set at a point where the fermentation tank becomes too full to allow effective fermentation to continue. In yet further embodiments, the particularly desired level can be flexibly set at a point when the fermentation tank is simply needed to make another fermentation run. The particular desired level of the cell density can be set by any one, two or all of the above-described factors.

In some methods of DNase degradation, any elevation of the pH and/or of the temperature after fermentation is assessed relative to the pH and temperature during fermentation. If the pH or temperature varies significantly (*i.e.*, more than 2°C or 0.5 pH units) over the period of fermentation, a mean value of the pH or temperature over the fermentation period is used as a baseline for comparison. Alternatively, particularly if the pH or temperature does not vary significantly then a single measurement can be used, *e.g.,* at the beginning or end of the fermentation period.

Preferably, the fermentation is conducted in such a way that the carbon-containing substrate is controlled as a limiting factor, thereby providing good conversion of the carbon-containing substrate to cells and avoiding contamination of the cells with a substantial amount of unconverted substrate. Unconverted substrates are not problematic if such substrates are water-soluble ones, however, because any remaining traces of such substrates are readily washed off. Nonetheless, added product-treatment steps such as suitable washing steps may be needed for non-water-soluble substrates.

Part or all of the carbon and energy source material, and/or part of the assimilable nitrogen source such as ammonia, can be added to the aqueous mineral medium before such a medium is fed to the fermenter.

Each of the streams introduced into the reactor preferably is controlled at a predetermined rate, or in response to a need determinable by monitoring such as concentration of the carbon and energy substrate, pH, dissolved oxygen, oxygen or carbon dioxide in the off-gases from the fermented, cell density measurable by light transmittance, or the like. The feed rates of the various materials can be varied so as to obtain as rapid a cell growth, rate as possible, consistent with efficient utilization of the carbon and energy source, to obtain as high a yield of microorganism cells relative to substrate charge as possible.

In either a batch, or the preferred fed batch operation, all equipment, reactor, or fermentation means, vessel or container, piping, attendant circulating or cooling devices, and the like, are initially sterilized, which can be accomplished by, for example, employing steam such as at about 121°C for an extended period, such as, for example, at least 15 min. The sterilized reactor then is inoculated with a culture of the selected microorganism in the presence of all the required nutrients, including oxygen, and the carbon-containing substrate.

The fermentation broth generally contains cellular debris, including cells, various suspended solids and other biomass contaminants including fungal host DNA, as well as the desired proteins, or proteins of interest. Preferably, at least about 40%, *e.g.*, at least about 50%, at least about 75%, or at least about 90% of the total amount expressed of each desired protein has been secreted into the broth at the end of the fermentation.

### IV. Reduction of DNA content of a fungal protein preparation

Once fermentation has produced a cell density or protein concentration as desired, the broth used in culturing or a protein preparation derived therefrom can then be processed to reduce its DNA content. It is believed that a typical fungal host organism or host cell (*e.g.*, *T. reesei, A. niger, A. tubingensis, A. oryzae, G. emersonii, M. thermophila, P. funiculosum, F. venenatum*, or *H. insolens*) can produce one or more DNase(s) that can be present in the cell culture broth after secretion or as a result of cell lysis, resulting in a DNA-degrading activity. The DNA content of a thus-made fungal protein preparation can be reduced exclusively or at least primarily by such endogenous DNase activity with none or insubstantial amount of exogenous DNase in the protein preparation.

At least one solid-liquid separation step can be performed to remove (*i.e.,* at least reduce the amount of) cells and cell debris from the broth before the removal of DNA. In some methods, after removal of cells and solids, proteins in the broth are concentrated, and sometimes further purified before the removal of DNA. However, any purification steps performed before incubation to remove DNA should preferably not separate the desired protein(s) from the DNase activity, and in any event not remove more than 10% (*e.g.,* no more than 20%, no more than 30%, no more than 40%, or no more than 50%) of the DNase activity. Similarly, any steps performed before incubation to remove DNA should preferably not inactivate the DNase activity, and in any event not inactivate more than 10% (*e.g.*, no more than 20%, no more than 30%, no more than 40%, or no more than 50%) of the DNase activity.

Cells and cellular debris can be removed by conventional solid-liquid separation techniques such as, *e*.*g*., centrifugation, filtration, dialysis, microfiltration, rotary vacuum filtration, or other known processes, to produce a cell-free filtrate. The fermentation broth or the cell-free filtrate can be further concentrated using techniques such as, for example, ultrafiltration, evaporation or precipitation. The proteinaceous components of the supernatant or filtrate can be precipitated by means of a salt, *e.g.,* ammonium sulfate, followed by purification by a variety of conventional purification procedures, such as ion exchange chromatography, affinity chromatography or similar conventional procedures.

To reduce the DNA content of a fungal protein preparation (*e.g.,* a *T*. *reesei*, *A. niger, A. tubingensis*, *A. oryzae*, *G. emersonii*, *M. thermophila*, *P. funiculosum*, *F venenatum*, or *H*. *insolens* protein preparation), the pH and/or the temperature of the protein preparation is adjusted, typically increased, so as to increase the fungal DNase activity in the preparation. The pH before the adjusting step is typically unchanged from that during fermentation. The temperature before the adjusting step typically ranges from the culturing temperature to room temperature depending on how long, if at all, the broth has cooled before the temperature is adjusted. The protein preparation is then incubated for a sufficient period at the adjusted pH and/or temperature to detectably decrease the DNA content and preferably reduce the DNA content to an undetectable level. In some methods, the adjustment in pH or temperature is an increase of the pH and/or temperature to above that used in the fermentation (typically at pH 4.0 - 5.0, and temperature 27-30°C). A combination of elevated pH and temperature values is effective but is not always necessary. For example, complete degradation of DNA can be achieved with an elevated pH alone, while the temperature of the protein preparation is kept low (*e.g.,* 10°C or 4°C), or maintained unchanged or at room temperature (*e.g.,* temperature within a range of 4°C -27° C). DNA can also be degraded with an elevated temperature alone, while the pH of the protein preparation is kept low (*e.g.*, pH 4-5) or maintained unchanged at that used in fermentation. Endogenous DNase activity in a fungal protein preparation can be detected using an assay such as those disclosed by, for example, Sinicropi, D., et al., (1994) Anal. Biochem. 222:351-358, or by Tolun, G. and Myers, R.S. (2003) Nucleic Acids Research 31: e111. Such an assay can be used to determine the optimum pH and/or temperature for endogenous DNase activity.

An elevated pH for DNA removal can be in the range of 5.0 to 9.0, and is preferably in the range of 6.0 to 8.0. Some examples of ranges of pH for DNA removal include 5.0 to 6.0, 5.2 to 7.8, 5.5 to 7.5, 6.0 to 7.0, 6.5 to 7.5, 7.0 to 8.0, and 8.0 to 9.0.

An elevated temperature for DNA removal can be in the range of 30° C to 70° C, and is preferably in the range of 35° C to 47° C. Some examples of ranges of temperature for DNA removal include 30° C to 40° C, 40° C to 50° C, 50° C to 60° C, and 60° C to 70° C. For thermophilic or thermostable proteins recombinantly expressed in a filamentous fungal host (*e.g.,* a *T. reesei*, *A. niger*, *A. tubingensis*, *A. oryzae*, *G. emersonii*, *M. thermophila*, *P. funiculosum*, *F. venenatum*, or *H. insolens*), a higher temperature can be used. The thermostability of the DNase of the particular fungal host is also determinative of such a higher temperature. For example, such a higher temperature may be no higher than about 66° C, such as no higher than about 60° C, 58° C, 55° C, or 52° C.

The protein preparation can be incubated at an elevated pH in combination with various ranges of temperature. For example, the pH can be elevated to the range of 5.2 to 7.8, 5.5 to 7.5, 5.5 to 6.5, 6.5 to 7.5, or 7.5 to 8.5 with a temperature maintained in the range of 0°C to 5°C, 5° C to 15° C, 15° C to 25° C, 25° C to 35° C, 35° C to 45° C, or 45° C to 55° C. Similarly, the protein preparation can be incubated at an elevated temperature in combination with various ranges of pH. For example, the temperature can be elevated to the range of 25° C to 35° C, 35° C to 45° C, or 45° C to 55° C with a pH maintained in the range of 3.5 to 4.5, 4.5 to 5.5, 5.5 to 6.5, 6.5 to 7.5, or 7.5 to 8.5.

If DNA removal by DNase is performed on an ultrafiltered concentrate of protein, the concentrate is typically at room temperature after ultrafiltration. In this case, the temperature can be increased above room temperature and/or the pH increased above the pH used in the fermentation of the fungal host.

The incubation time for DNA removal during the adjusting step can vary considerably, depending in part on the degree of reduction of DNA content desired, and the sensitivity of the desired proteins to the temperature and pH used for DNA removal. Removal of DNA to an undetectable level is preferred. Also preferred is little or no loss of activity of desired protein(s). Preferably, the optimized incubation conditions result in no loss or insignificant loss of protein stability or activity, *e*.*g*., less than 1%, 2%, 5%, 10%, 15%, or 20% loss of activity of desired protein(s). Optionally, protein stability or activity can be assessed before and/or after the incubation. The time period for the DNA removal incubation also depends on the pH and/or temperature during DNA removal, a longer time being required as the pH moves to below 7 and the temperature moves to below 40° C. The time period is generally at least 10, 20, 30, 60, 120, 180, 240 min and up to 4, 6, 12, 24 or 48 hr including all permutations of lower and upper limits. The incubation time can also be longer than 48 hr. A preferred incubation time is 2-8 hr. Preferably, the pH and/or temperature adjusting step is performed before the enzyme(s) produced by the host cell is/are applied to treat or act on an intended substrate.

The DNA content of the protein preparation (e.g., the culture broth) can also be assessed before and after incubation of the adjusting step. Any segment of fungal host DNA can be used as a marker of the total DNA contents of the preparation. Preferably a genomic segment is used. Optionally more than one segment can be detected.

PCR amplification is a suitable and preferred method for analyzing nucleic acid (e.g., DNA) content. Primers can be designed to flank any suitable genomic DNA segment (for example, the entire genomic sequence of *T. reesei* can be obtained from the website of the U.S. Department of Energy, Joint Genome Institute, and the entire genomic sequence of *Aspergillus* sp. can be obtained from the *Aspergillus* genome database hosted by University of Maryland School of Medicine and the Department of Genetics at the School of Medicine, Stanford University, as well as from the fungal genome initiative website of the Broad Institute). PCR detection can be qualitative or quantitative. Performing amplification followed by detection of the amplification product, if any, by ethidium bromide staining of a gel would indicate the presence or absence of the underlying marker but does not provide an accurate measurement of the amount of such marker. Presence of DNA can also be detected by gel electrophoresis and ethidium bromide staining without PCR amplification. DNA is shown by a characteristic smear or ladder of bands.

In a quantitative amplification, the amount of amplification product is proportional to the amount of template in the original sample and detection occurs in real time. Comparison to appropriate controls provides a measure of the copy number of the DNA segment being amplified. Detailed protocols for quantitative PCR are provided in Innis et al. (1990) PCR Protocols, A Guide to Methods and Applications, Academic Press, Inc. N.Y.

Other suitable amplification methods for use in detection include ligase chain reaction (LCR), (see Wu and Wallace (1989) Genomics 4: 560, Landegren et al. (1988) Science 241: 1077, and Barringer et al. (1990) Gene 89: 117, transcription amplification (Kwoh et al. (1989) Proc. Natl. Acad. Sci. USA 86: 1173), self-sustained sequence replication (Guatelli et al. (1990) Proc. Nat. Acad. Sci. USA 87: 1874), dot PCR, and linker adapter PCR.

The presence or the level of DNA in the protein preparation (e.g., the culture broth) can also be assessed by, for example, hybridization-based assays. Methods such as Southern blotting are described in *e.g.,* Sambrook et al., (1989) Molecular Cloning: A Laboratory Manual (2d Ed., vols 1-3, Cold Spring Harbor Press, New York). Commercial DNA assay kits are also available, such as the Quant-iT™ DNA assay kit supplied by InVitrogen Corporation (Carlsbad, CA).

Treatment of a broth or other protein preparation using the present methods results in detectable reduction in DNA, and preferably the DNA content is reduced to an undetectable level. A level is considered undetectable if PCR amplification of any segment of genomic DNA present in a single copy in a haploid genome followed by ethidium bromide staining gives no visible band (using the PCR conditions as in the present Examples). In an example of a typical regulatory requirement, "no detectable DNA in the final product" may be ascertained using a PCR-based assay with a detection limit of, for example, 1 to 5 ng of total filamentous fungal DNA/mL enzyme preparation. In another example, depending on the formulation and/or use of the final product, detection limits of total filamentous fungal DNA of 20 pg/mL, or 2 ng/mL, or 10 ng/mL can be imposed. In certain other examples, an even lower limit of about 500 femtogram total filamentous fungal DNA/g of lyophilized product (e.g., less than about 450 fg of DNA per g of lyophilized product, less than about 400 fg/g, less than about 350 fg/g, less than about 300 fg/g, less than about 250 fg/g, or even less than about 200 fg/g) can be achieved using the present method, for example, by extending the incubation time at the elevated temperature and/or at the elevated pH. In addition, the use of the instant method in combination with conventional methods of removing DNA from culture broths or other protein preparation is also contemplated. For example, the herein-described method can be used to reduce the level of DNA in the culture broth, followed by the addition of small amounts of exogenous DNase so as to further removal residual DNA from such a broth, provided that the contribution of such exogenous DNase to degradation is no more than 49% (e.g., no more than 49%, no more than 45%, no more than 40%, no more than 35%, no more than 30%, no more than 25%, no more than 20%, no more than 15%, no more than 10%, or no more than 5%) of the total DNase activity.

After removing DNA from a protein preparation (including, e.g., a culture broth), the temperature if elevated during DNA removal can then be allowed to fall to a lower temperature, *e.g.,* room temperature, or cooled to a lower temperature of 4° C or other temperature suitable for storage of the preparation. It is usually not necessary to re-adjust the pH of the preparation to a different value. However, if a downstream formulation or other procedures require a particular pH, the pH of the preparation can be adjusted to the desired value.

Because fermentation products or broth of other recombinant filamentous host cells can be expected to contain some level of endogenous DNase activity, just as in the *T*. *reesei* culture broth, the method described herein can be used with equal facility to remove DNA from such broths or other protein preparations. It can be expected that the level of DNase activity and the pH or temperature optima of the endogenous DNases may vary from species to species, a skilled person can ascertain the pH adjustment and/or temperature adjustment, as well as the incubation period required to remove the DNA molecules using the teachings provided here without extensive experimentation.

### V. Formulation

After the fungal host DNA of the protein preparation (including, e.g., a culture broth) has been degraded, the preparation can be packaged for sale or used essentially as is with minimal, if any, further processing, or the protein preparation can be further purified or otherwise processed and/or formulated for downstream applications. Protein(s) or protein preparations can be formulated, for example, as a liquid, a slurry, a powder, a spray, a suspension, a lyophilized composition/formulation, a solid, granule, geltab, pill, implant, a gel; or a pharmaceutical formulation, a food, a feed, a food supplement, a feed supplement, a food additive, a feed additive, a nutritional supplement or a dietary supplement thereof.

### VI. Uses of proteins

Protein(s) or protein preparations (*e*.*g*., a broth) generated by the present methods have agricultural, industrial, medical and nutritional applications. For example, cellulase enzymes can be used for generating glucose from grain, or as a supplement in animal feed to decrease the production of fecal waste by increasing the digestibility of the feed. Cellulase enzymes can also be used to increase the efficiency of alcoholic fermentations (*e.g.,* in beer brewing) by converting lignocellulosic biomass into fermentable sugars. Phytase preparation can be used in grain wet milling, animal feed and cleaning products. Phytases, phytate and lower phosphate phytate derivatives also find many other uses in personal care products, medical products and food and nutritional products, as well as in various industrial applications, particularly in the cleaning, textile, lithographic and chemical arts. Lipases can be used in various food/feed, baking, cleaning, and/or biofuels applications. Proteins such as hormones, growth factors, cytokines, and antibodies can be used in treatment and prophylaxis of disease.

### EXAMPLES

### Example 1

### Part A:

A recombinant *T. reesei* strain expressing a phytase from *Buttiauxella* sp. was cultured in a bioreactor. The expression cassette producing the phytase was prepared in accordance with the protocols described in U.S. Patent Publication US2009/0098249. The expression cassette was then inserted into a host strain that was derived from a "Quad deletion" version (wherein four major secreted cellulases CBHI (cel7a), CBHII (cel6a), EGI (cel7b), EGII (cel5a) have been deleted) of *T. reesei* strain RL-P37 (Sheir-Neiss and Montenecourt, 1984, Appl. Microbiol. Biotechnol. 20:46-53). The bioreactor was operated as described in Patent publication US20040121446.

Following growth in the bioreactor the culture supernatant was obtained by filtration to remove the *T*. *reesei* cells. The supernatant was concentrated approximately four-fold by ultrafiltration. Sodium benzoate and potassium sorbate were added, both to a final concentration of 0.3%, and pH was adjusted to 5.5 to provide the ultrafiltration concentrate (UFC) used in the experiments below.

A 1,526 bp fragment of DNA (the spike DNA) was obtained by polymerase chain reaction (PCR) and purified using a Qiagen (Valencia, CA) PCR purification kit according to the manufacturer's directions. This spike DNA was added to UFC samples at a final concentration of 1 µg/mL. The oligonucleotide primers used in the PCR could be used to generate the same fragment of DNA using as template either genomic DNA from the *T. reesei* strain that was grown in the bioreactor or a purified plasmid DNA containing the same template DNA sequence. The DNA sequence of the spike DNA is not important. Any region of the *T. reesei* genomic DNA can be used as template and primers designed by conventional procedures.

Procedure to test for host DNA in UFC samples: DNA was purified from 50 µL samples of UFC (to which a spike DNA preparation was added if desired) using a Promega (Madison, WI) Wizard SV Gel and PCR Clean-Up System according to the manufacturer's directions. Briefly, the 50 µL UFC sample was mixed with 450 µL ofPromega Membrane Binding Solution and this mix was loaded onto a Wizard SV Minicolumn. After washing the minicolumn with Membrane Wash Solution any DNA bound to the minicolumn membrane was finally eluted in nuclease-free water. Detection of DNA in the eluted sample was by PCR using the same primers used to generate the spike DNA followed by visualization of DNA fragments by agarose gel electrophoresis and staining with ethidium bromide. Two DNA bands were often observed in the PCR products, presumably due to some non-specific binding of the primers

### Part B:

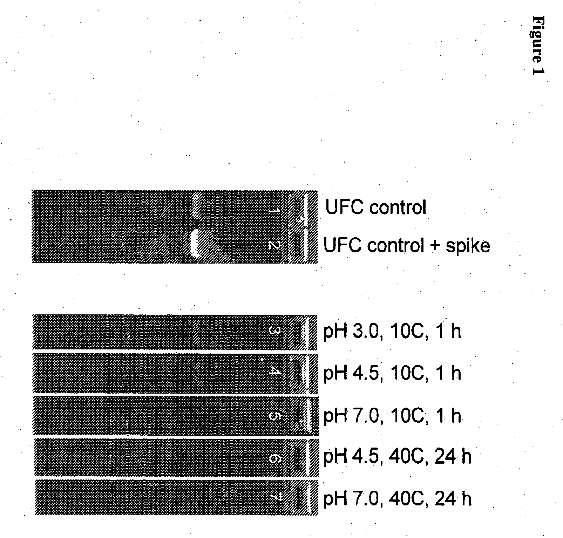

A UFC sample was selected that contained detectable DNA by PCR analysis without the addition of spike DNA (Figure 1, lane 1). Spike DNA was added to this UFC to a final concentration of 1 µg/mL and the amount of PCR product obtained was seen to increase (Figure 1, lane 2). The UFC (without spike DNA) was adjusted to pH 3.0, pH, 4.5 or pH 7.0 and incubated at 10° C for 1 hr. DNA remained detectable by PCR analysis after incubation at pH 3.0 and pH 4.5 but not after incubation at pH 7.0. The UFC (without spike DNA) was adjusted to pH, 4.5 or pH 7.0 and incubated at 40° C for 24 hr following which DNA was undetectable by PCR analysis.

### Part C:

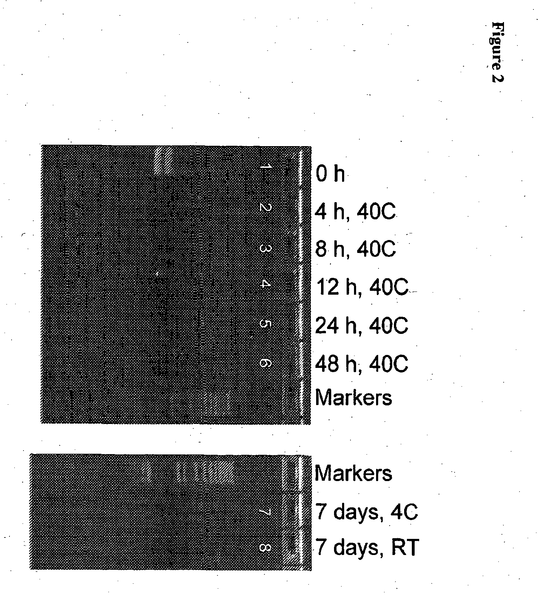

Spike DNA was added at a final concentration of 1 µg/mL to a UFC sample at pH 5.5. The sample was incubated at 40° C for 0, 4, 8, 12, 24, or 48 hr. With no incubation the spike DNA was detectable by PCR analysis (Figure 2, lane 1). However, by 4 hr of incubation the spike DNA was no longer detectable (Figure 2, lane 2). No loss of phytase activity was observed during incubation up to 24 hr. The same UFC sample with spike DNA was incubated for 7 days at 4° C or room temperature after which no DNA was detectable by PCR analysis (Figure 2, lanes 7 and 8).

### Part D:

Spike DNA was added at a final concentration of 1.45 µg/mL to a UFC sample at pH 5.5. The sample was incubated at 10° C or 22° C or 37° C for 0, 4, 8, 12, or 24 hr followed by PCR analysis to detect the spike DNA.

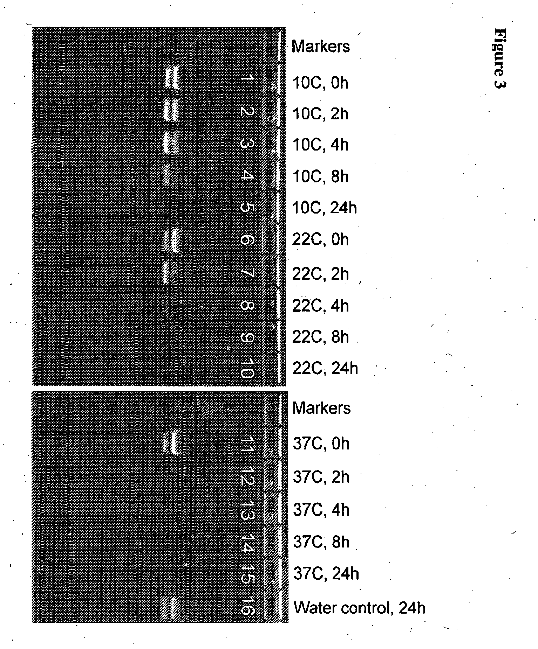

After incubation for 8 hr at 10° C DNA was detectable by PCR (Figure 3, lane 4), and even after 24 hr at 10° C a trace of PCR product was observed (Figure 3, lane 5). After incubation for 8 hours at 22° C DNA was detectable by PCR (Figure 3, lane 9), but after 24 hr at 22° C no PCR product was observed (Figure 3, lane 10). After incubation for 2 or 4 hr at 37° C a trace of PCR product was observed (Figure 3, lanes 12 and 13), but after 8 hr at 37° C no DNA was detected (Figure 3, lane 14). A control experiment consisting of the spike DNA in water clearly showed detectable DNA after incubation for 24 hr at 37° C confirming that loss of DNA was due to components in the UFC (Figure 3, lane 16).

### Example 2

A recombinant *T. reesei* strain expressing a lipase from *Aspergillus tubingensis* was cultured in a bioreactor. The expression cassette producing the lipase was prepared in accordance with the protocols described in PCT Patent publication WO 2010/122531. The expression cassette was then inserted into a host strain that was derived from a "Quad deletion" version (wherein four major secreted cellulases CBHI (cel7a), CBHII (cel6a), EGI (cel7b), EGII (cel5a) have been deleted) of *T. reesei* strain R.L-P37 (Sheir-Neiss and Montenecourt, 1984, Appl. Microbiol. Biotechnol. 20:46-53). The bioreactor was operated as described in Patent publication US20040121446.

Following growth in the bioreactor the culture supernatant was obtained by filtration to remove the *T*. *reesei* cells. The supernatant was concentrated approximately four-fold by ultrafiltration to create the ultrafiltration concentrate (UFC) used for further experiments.

A Promega (Madison, WI) Wizard SV Gel and PCR Clean-Up System was used according to the manufacturer's instructions to extract DNA from UFC samples. Briefly, 100 µL UFC sample was mixed with 450 µL of Promega Membrane Binding Solution and this mix was loaded onto a Wizard SV Minicolumn. After washing the minicolumn with Membrane Wash Solution any DNA bound to the minicolumn membrane was finally eluted in nuclease-free water. Detection of DNA in the eluted sample was by agarose gel electrophoresis and staining with ethidium bromide. A smear of low molecular weight DNA fragments (approximately 300 bp and smaller) was often observed in UFC samples and is presumably derived from fragmented *T. reesei* genomic DNA.

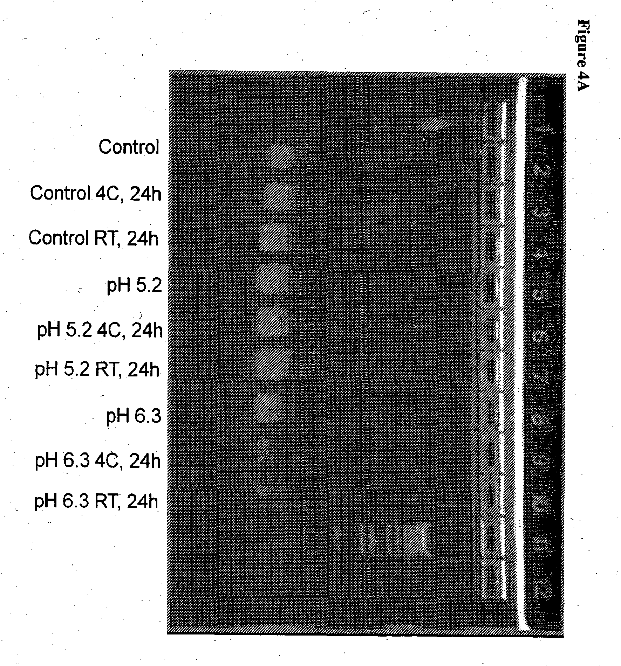

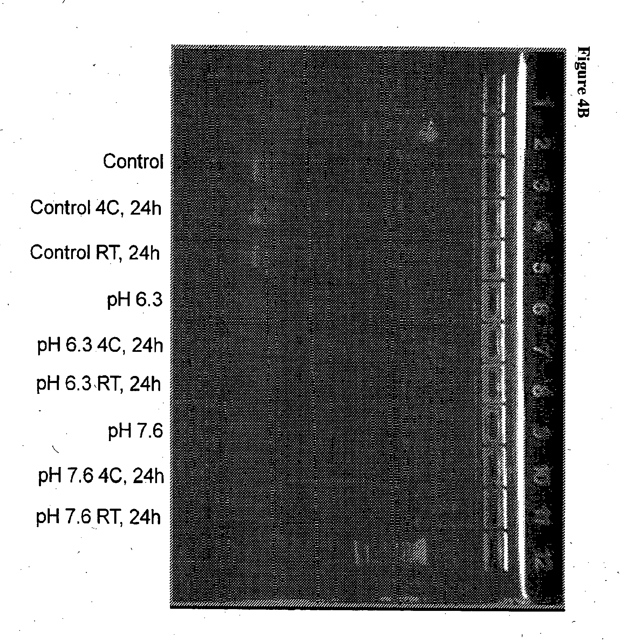

Various treatments of the UFC were tested and their effects on the abundance of DNA recovered from the UFC were observed. The starting pH of the UFC was approximately 4.0 (the control UFC). Samples of UFC were adjusted to pH 5.2, 6.3 or 7.6. The control UFC and the adjusted UFCs were either frozen immediately after pH adjustment or were incubated for 24 hr at 4° C or room temperature and frozen until further analysis. All samples were then analyzed by agarose gel electrophoresis. The results are shown in Figures 4A and 4B. Incubation of then control UFC or the UFC adjusted to pH 5.2 had no obvious effect on the presence of DNA. Incubation of UFC adjusted to pH 6.3 caused a slight reduction in DNA. Incubation of UFC adjusted to pH, 7.6 caused an obvious reduction in DNA such that after 24 hr at room temperature the DNA smear was undetectable by agarose gel electrophoresis.

Although preferred methods and materials have been described, any methods and materials similar or equivalent to those described herein can be used in the practice or testing of the present invention. Unless otherwise apparent from the context, any embodiment, aspect, step, feature, element or limitation can be used in combination with any other.

## Claims

1. A method of reducing DNA content of a broth in which filamentous fungal host cells has been cultured, comprising the steps of:
performing a solid-liquid separation step to separate the broth from filamentous fungal host cells before adjusting the pH and/or temperature of a broth in which the fungal host cells have been cultured for at least 24 hours to increase the pH and/or the temperature used in culturing;
incubating the broth for a sufficient period at the increased pH and/or temperature to detectably reduce fungal host DNA in the preparation; and assessing the DNA content of the broth before and after the incubating step;
wherein the reduction of DNA content is primarily due to the presence of endogenous DNase in the broth.

2. The method of claim 1, further comprising an ultrafiltration step, wherein the macromolecules in the broth are concentrated by performing ultrafiltration before the adjusting step.

3. The method of claim 2, wherein the broth is at room temperature after the ultrafiltration step.

4. The method of any one of claims 1-3, wherein:
(a) the temperature of the broth before the adjusting step is 25°C to 34°C; and/or
(b) the pH of the broth before the adjusting step is between 4 and 5.

5. The method of any one of claims 1-4, further comprising culturing the filamentous fungal host cells in the broth until a desired concentration of secreted proteins of interest in the broth is obtained before the adjusting step.

6. The method of any one of claims 1-5, wherein:
(a) the pH is increased to pH 6-8 during the adjusting step; and/or
(b) the temperature is increased to 35°C to 47°C during the adjusting step.
determining a reduction of the amount of filamentous fungal host cell DNA in the protein preparation;
wherein the reduction of DNA content is primarily due to the presence of endogenous DNase in the broth.

7. The method of any one of claims 1-6, wherein the DNA content is reduced to an undetectable level assessed by PCR and/or gel electrophoresis with ethidium bromide staining.

8. The method of any one of claims 1-6, further comprising allowing the broth to cool to room temperature after the incubating step and/or purifying one or more proteins from the broth.

9. The method of any one of claims 1-6, wherein one or more proteins in the broth are recombinantly expressed by the filamentous fungal host cell.

10. The method of claim 9, wherein the filamentous fungal host cell lacks an exogenous DNase.

11. The method of any one of claims 1-10, wherein no DNase is added to the broth.

12. The method of any one of claims 1-10, wherein the filamentous fungal host cell recombinantly expresses a cellulase enzyme.

13. The method of any one of claims 1-11, wherein the filamentous fungal host cell recombinantly expresses a phytase or a lipase.

14. A method of reducing DNA content of a protein preparation from filamentous fungal ost cells, comprising the steps of:
assessing a level of filamentous fungal host cell DNA in a protein preparation from the fungal host cells;
performing a solid-liquid separation step to separate the protein preparation from the filamentous fungal host cells before increasing the pH and/or temperature of the protein preparation to an adjusted pH and/or temperature;
incubating the protein preparation for a sufficient period at the adjusted pH and/or temperature to detectably reduce the level of filamentous fungal host cell DNA in the protein preparation; and

15. The method of claim 14, wherein the amount of the DNA has been reduced to an undetectable level.

16. The method of any one of claims 1-15, wherein the filamentous fungal host cell is a cell of *T*. *reesei, A. niger, A. tubingensis, A. oryzae, G. emersonii, M. thermophila, P. funiculosum, F. venenatum, or H. insolens* .

## Patentansprüche

1. Verfahren zum Reduzieren eines DNA-Inhalts einer Bouillon, in der filamentöse pilzartige Wirtszellen kultiviert wurden, die folgenden Schritte umfassend:
Durchführen eines Feststoff-Flüssigkeit-Trennungsschritts, um die Bouillon von den filamentösen pilzartigen Wirtszellen zu trennen, vor dem Anpassen des pH-Werts und/oder der Temperatur einer Bouillon, in der die pilzartigen Wirtszellen mindestens 24 Stunden kultiviert wurden, um den pH-Wert und/oder die Temperatur, der/die beim Kultivieren verwendet wurden, zu erhöhen;
Inkubieren der Bouillon für eine ausreichende Zeitdauer bei dem/der erhöhten pH-Wert und/oder Temperatur, um pilzartige Wirts-DNA in der Präparation detektierbar zu reduzieren; und Bewerten des DNA-Inhalts der Bouillon vor und nach dem Schritt des Inkubierens;
wobei die Reduktion des DNA-Inhalts hauptsächlich auf der Gegenwart von endogener DNase in der Bouillon beruht.

2. Verfahren nach Anspruch 1, ferner umfassend einen Ultrafiltrationsschritt, wobei die Makromoleküle in der Bouillon vor dem Anpassungsschritt durch Durchführen von Ultrafiltration konzentriert werden.

3. Verfahren nach Anspruch 2, wobei die Bouillon nach dem Ultrafiltrationsschritt auf Raumtemperatur ist.

4. Verfahren nach einem der Ansprüche 1-3, wobei:
(a) die Temperatur der Bouillon vor dem Anpassungsschritt 25°C bis 34°C beträgt; und/oder
(b) der pH-Wert der Bouillon vor dem Anpassungsschritt zwischen 4 und 5 liegt.

5. Verfahren nach einem der Ansprüche 1-4, ferner umfassend, vor dem Anpassungsschritt, Kultivieren der filamentösen pilzartigen Wirtszellen in der Bouillon, bis eine gewünschte Konzentration von interessierenden sekretierten Proteinen in der Bouillon erhalten wird.

6. Verfahren nach einem der Ansprüche 1-5, wobei:
(a) der pH-Wert während des Anpassungsschritts auf pH 6-8 erhöht wird; und/oder
(b) die Temperatur während des Anpassungsschritts auf 35°C bis 47°C erhöht wird.

7. Verfahren nach einem der Ansprüche 1-6, wobei der DNA-Inhalt auf ein nicht detektierbares Niveau reduziert wird, bewertet durch PCR und/oder Gelelektrophorese mit Ethidiumbromid-Anfärbung.

8. Verfahren nach einem der Ansprüche 1-6, ferner umfassend, die Bouillon nach dem Inkubationsschritt auf Raumtemperatur abkühlen zu lassen und/oder ein oder mehrere Proteine aus der Bouillon zu reinigen.

9. Verfahren nach einem der Ansprüche 1-6, wobei ein oder mehrere Proteine in der Bouillon durch die filamentöse pilzartige Wirtszelle rekombinant exprimiert werden.

10. Verfahren nach Anspruch 9, wobei der filamentösen pilzartigen Wirtszelle eine exogene DNase fehlt.

11. Verfahren nach einem der Ansprüche 1-10, wobei der Bouillon keine DNase hinzugefügt wird.

12. Verfahren nach einem der Ansprüche 1-10, wobei die filamentöse pilzartige Wirtszelle ein Cellulaseenzym rekombinant exprimiert.

13. Verfahren nach einem der Ansprüche 1-11, wobei die filamentöse pilzartige Wirtszelle eine Phytase oder eine Lipase rekombinant exprimiert.

14. Verfahren zum Reduzieren des DNA-Inhalts einer Proteinpräparation aus filamentösen pilzartigen Wirtszellen, die folgenden Schritte umfassend:
Bewerten eines Niveaus filamentöser pilzartiger Wirtszellen-DNA in einer Proteinpräparation aus den pilzartigen Wirtszellen;
Durchführen eines Feststoff-Flüssigkeit-Trennungsschritts, um die Proteinpräparation von den filamentösen pilzartigen Wirtszellen zu trennen, vor dem Erhöhen des pH-Werts und/oder der Temperatur der Proteinpräparation auf einen angepassten pH-Wert und/oder eine angepasste Temperatur;
Inkubieren der Proteinpräparation für eine ausreichende Zeitdauer bei dem/der angepassten pH-Wert und/oder Temperatur, um das Niveau von filamentösen pilzartigen Wirtszellen-DNA in der Proteinpräparation detektierbar zu reduzieren; und
Bestimmen einer Reduktion der Menge der DNA von filamentösen pilzartigen Wirtszellen in der Proteinpräparation;
wobei die Reduktion des DNA-Inhalts hauptsächlich auf der Gegenwart von endogener DNase in der Proteinpräparation beruht.

15. Verfahren nach Anspruch 14, wobei die Menge der DNA auf ein nicht detektierbares Niveau reduziert wurde.

16. Verfahren nach einem der Ansprüche 1-15, wobei die filamentöse pilzartige Wirtszelle eine Zelle von *T. reesei, A. niger, A. tubingensis, A. oryzae, G. emersonii, M. thermophila, P. funiculosum, F. venenatum* oder *H. insolens* ist.

## Revendications

1. Méthode pour la réduction de la teneur en ADN d'un bouillon dans lequel des cellules hôtes de champignon filamenteux ont été cultivées, comprenant les étapes:
de réalisation d'une étape de séparation solide-liquide pour séparer le bouillon des cellules hôtes de champignon filamenteux avant l'étape d'ajustement du pH et/ou la température d'un bouillon dans lequel les cellules hôtes de champignon ont été cultivées pendant au moins 24 heures pour augmenter le pH et/ou la température utilisés dans la culture ;
d'incubation du bouillon pendant une période suffisante au pH et/ou à la température augmentés pour réduire de façon détectable l'ADN de l'hôte fongique dans la préparation; et d'évaluation de la teneur en ADN du bouillon avant et après l'étape d'incubation;
dans laquelle la réduction de la teneur en ADN est principalement due à la présence d'une DNase endogène dans le bouillon.

2. Méthode selon la revendication 1, comprenant en outre une étape d'ultrafiltration, dans laquelle les macromolécules dans le bouillon sont concentrées par la réalisation de l'ultrafiltration avant l'étape d'ajustement.

3. Méthode selon la revendication 2, dans laquelle le bouillon est à la température ambiante après l'étape d'ultrafiltration.

4. Méthode selon l'une quelconque des revendications 1-3, dans laquelle:
(a) la température du bouillon avant l'étape d'ajustement est de 25°C à 34°C; et/ou
(b) le pH du bouillon avant l'étape d'ajustement est entre 4 et 5.

5. Méthode selon l'une quelconque des revendications 1-4, comprenant en outre la culture des cellules hôtes de champignon filamenteux dans le bouillon jusqu'à ce qu'une concentration désirée de protéines sécrétées intéressantes dans le bouillon soit obtenue avant l'étape d'ajustement.

6. Méthode selon l'une quelconque des revendications 1-5, dans laquelle:
(a) le pH est augmenté à pH 6-8 durant l'étape d'ajustement; et/ou
(b) la température est augmentée à 35°C à 47°C durant l'étape d'ajustement.

7. Méthode selon l'une quelconque des revendications 1-6, dans laquelle la teneur en ADN est réduite à un niveau non détectable évalué par une PCR et/ou une électrophorèse sur gel avec une coloration au bromure d'éthidium.

8. Méthode selon l'une quelconque des revendications 1-6, consistant en outre à laisser le bouillon refroidir à la température ambiante après l'étape d'incubation et/ou à purifier une ou plusieurs protéines à partir du bouillon.

9. Méthode selon l'une quelconque des revendications 1-6, dans laquelle une ou plusieurs protéines dans le bouillon sont exprimées par recombinaison par la cellule hôte de champignon filamenteux.

10. Méthode selon la revendication 9, dans laquelle la cellule hôte de champignon filamenteux est sans DNase exogène.

11. Méthode selon l'une quelconque des revendications 1-10, dans laquelle il n'est pas ajouté de DNase dans le bouillon.

12. Méthode selon l'une quelconque des revendications 1-10, dans laquelle la cellule hôte de champignon filamenteux exprime par recombinaison une enzyme cellulase.

13. Méthode selon l'une quelconque des revendications 1-11, dans laquelle la cellule hôte de champignon filamenteux exprime par recombinaison une phytase ou une lipase.

14. Méthode pour la réduction de la teneur en ADN d'une préparation de protéine provenant de cellules hôtes de champignon filamenteux, comprenant les étapes:
d'évaluation d'un niveau d'ADN de cellules hôtes de champignon filamenteux dans une préparation de protéine provenant des cellules hôtes fongiques;
de réalisation d'une étape de séparation solide-liquide pour séparer la préparation de protéine des cellules hôtes de champignon filamenteux avant d'augmenter pH et/ou la température de la préparation de protéine jusqu'à atteindre un pH et/ou une température ajustés;
d'incubation de la préparation de protéine pendant une période suffisante au pH et/ou à la température ajustés pour réduire de façon détectable le niveau d'ADN de cellules hôtes de champignon filamenteux dans la préparation de protéine; et
de détermination d'une réduction de la quantité d'ADN de cellules hôtes de champignon filamenteux dans la préparation de protéine;
dans laquelle la réduction de la teneur en ADN est principalement due à la présence de DNase endogène dans le bouillon.

15. Méthode selon la revendication 14, dans laquelle la quantité de l'ADN a été réduite à un niveau non détectable.

16. Méthode selon l'une quelconque des revendications 1-15, dans laquelle la cellule hôte de champignon filamenteux est une cellule de *T. reesei, A. niger, A. tubingensis, A. oryzae, G. emersonii, M. thermophila, P. funiculosum, F. venenatum* ou *H. insolens.*
